# EUROPÄISCHE PATENTSCHRIFT

(11) **EP 0 780 367 B1**
(45) Veröffentlichungstag und Bekanntmachung des Hinweises auf die Patenterteilung: **03.11.1999**
(21) Anmeldenummer: 96119756.3
(22) Anmeldetag: 10.12.1996
(51) Int. Cl.: C07C 303/24, C07C 305/10, C07C 305/06, C11D 1/12, C11D 1/29

(54) **Anionische Detergensgemische**
Anionic detergent mixture
Mélange de détergent anionique

(30) Priorität: 19.12.1995 DE 19547458
(43) Veröffentlichungstag der Anmeldung: 25.06.1997
(73) Patentinhaber: Henkel Kommanditgesellschaft auf Aktien, 40589 Düsseldorf-Holthausen (DE)
(72) Erfinder: Raths, Hans-Christian, Dr., 40789 Monheim (DE); Rüben, Rainer, 40789 Monheim (DE)

(56) Entgegenhaltungen:
- DE-A- 4 137 221
- DE-B- 1 195 301
- US-A- 4 116 986
- DATABASE WPI Section Ch, Week 8340 Derwent Publications Ltd., London, GB; Class D25, AN 83-781677 XP002033951 & SU 979 334 A (KHARKOV POLY) , 7.Dezember 1982
- "RÖMPP Chemie Lexikon", 9. Auflage, Band 2, 1993, GEORG THIEME VERLAG, STUTTGART, NEW YORK, S. 1337, 1343
- The Condensed Chemical Dictionary, 9th edition, 1997, Van Nostrand Reinhold, S. 374

## Beschreibung

Die Erfindung betrifft anionische Detergensgemische, die man durch Co-Sulfatierung von Fettsäurealkanolamiden und Fettalkoholalkoxylaten erhält, ein Verfahren zu ihrer Herstellung sowie ihre Verwendung zur Herstellung von oberflächenaktiven Mitteln.

### Stand der Technik

Fettsäurealkanolamidsulfate stellen milde anionische Tenside dar, deren Herstellung und Eigenschaften seit langem bekannt sind. Übersichten hierzu finden sich beispielsweise von J.K. Weil et al. in **J. Am. Oil. Chem. Soc. 47, 91 (1970)** und **ibid. 48, 35 (1971)**. Die Herstellung erfolgt durch Anlagerung von Schwefelsäure, Schwefeltrioxid, Chlorsulfonsäure oder einem anderen Sulfatierungsmittel an die freie Hydroxylgruppe der Aminoalkoholkomponente. Entsprechende Verfahren werden beispielsweise in den Druckschriften **DE-AS 11 95 301** (Henkel), **DE-A1 41 37 221** (Henkel), **NL-A 125 242** (Marchon) sowie den US-Patenten **US 1,932,180 (IG Farben), US 1,981,792** (Orelup) und **US 2,551,125** (Procter & Gamble) beschrieben.

Infolge ihres hohen Schmelzpunktes ist es jedoch erforderlich, die Sulfatierung entweder im Lösungsmittel oder bei sehr hohen Temperaturen durchzuführen. Während die Mitverwendung von inerten Lösungsmitteln nicht gewünscht wird, da zu ihrer nachträglichen Abtrennung ein hoher technischer Aufwand erforderlich ist, ist die Sulfatierung bei hohen Temperaturen von Nachteil, da Alkanolamide unter diesen Bedingungen leicht Wasser abspalten und Oxazoline bilden. Aus diesen Gründen werden die Fettsäurealkanolamide üblicherweise vor der Sulfatierung mit Ethylenoxid umgesetzt; die Ethoxylate weisen einen niedrigeren Schmelzpunkt auf und lassen sich daher auch bei milderen Bedingungen in die Ethersulfate überführen.

Aus dem US-Patent **US 4,116,986** und der korrespondierenden Veröffentlichung von R.G. Bistline et al. in **J. Am. Oil. Chem. Soc. 54, 371 (1977)** ist ein Verfahren zur Herstellung von sulfatierten Fettsäurealkanolamiden bekannt, bei dem man die Sulfatierung in Gegenwart niederer Alkohole, vorzugsweise Isopropylalkohol durchführt. Die resultierenden Sulfatgemische besitzen jedoch ein unzureichendes Schaum- und Reinigungsvermögen.

Die Aufgabe der Erfindung hat somit darin bestanden, ein Verfahren zur Sulfatierung von Fettsäurealkanolamiden zur Verfügung zu stellen, welches bei milden Bedingungen hellfarbige, schaum- und reinigungsstarke Aniontenside mit vermindertem Gehalt an Oxazolinen zur Verfügung stellt.

### Beschreibung der Erfindung

Gegenstand der Erfindung sind anionische Detergensgemische, die man erhält, indem man Mischungen von
(a) Fettsäurealkanolamiden mit 6 bis 22 Kohlenstoffatomen im Fettsäurerest und
(b) Fettalkoholpolyalkylenglycolen mit 6 bis 22 Kohlenstoffatomen im Fettalkoholrest
im Gewichtsverhältnis 5 : 95 bis 70 : 30, vorzugsweise 20 : 80 bis 50 : 50, in an sich bekannter Weise gemeinsam sulfatiert und anschließend mit wäßrigen Basen neutralisiert.

Überraschenderweise wurde gefunden, daß die gemeinsame Sulfatierung der Komponenten (a) und (b) problemlos möglich ist und zu schaum- und reinigungsstarken Detergensgemischen führt, die sich zudem durch einen vergleichsweise niedrigen Gehalt an unerwünschten Oxazolinverbindungen auszeichnen.

Ein weiterer Gegenstand der Erfindung betrifft ein Verfahren zur Herstellung von anionischen Detergensgemischen, bei dem man Mischungen von
(a) Fettsäurealkanolamiden mit 6 bis 22 Kohlenstoffatomen im Fettsäurerest und
(b) Fettalkoholpolyalkylenglycolen mit 6 bis 22 Kohlenstoffatomen im Fettalkoholrest
im Gewichtsverhältnis 5 : 95 bis 70 : 30, vorzugsweise 20 : 80 bis 50 : 50, in an sich bekannter Weise gemeinsam sulfatiert und anschließend mit wäßrigen Basen neutralisiert.

### Fettsäurealkanolamide

Fettsäurealkanolamide stellen bekannte Stoffe dar, die nach den einschlägigen Methoden der präparativen organischen Chemie, beispielsweise durch Amidierung von Fettsäuren mit 6 bis 22 Kohlenstoffatomen mit Aminoalkoholen hergestellt werden können. Die Fettsäurealkanolamide, die die Komponente (a) bilden, folgen vorzugsweise der Formel **(I)**,

N¹CO-NH(CH₂)ₙOH (I)

in der R¹CO für einen linearen oder verzweigten gesättigten und/oder ungesättigten Acylrest mit 6 bis 22 Kohlenstoffatomen und n für Zahlen von 2 bis 4 steht. Typische Beispiele sind Amidierungsprodukte von Ethanolamin und/oder Propanolamin mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind Laurinsäureethanolamid sowie Ethanolamide auf Basis von technischen Kokosfettsäureschnitten mit 8 bis 18 bzw. 12 bis 14 Kohlenstoffatomen.

### Fettalkoholpolyalkylenglycolether

Auch Fettalkoholpolyalkylenglycolether stellen bekannte Stoffe dar, die man großtechnisch durch alkalikatalysierte Anlagerung von Ethylenoxid und/oder Propylenoxid an primäre Alkohole mit 6 bis 22 Kohlenstoffatomen erhält. Die Fettalkoholpolyalkylenglycolether, die im Sinne der Erfindung die Komponente (b) bilden, folgen der Formel **(II)**, 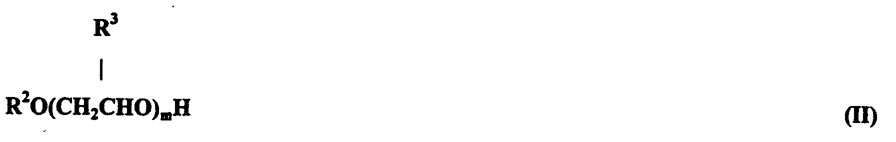 in der R² für einen linearen Alkyl- und/oder Alkenylrest mit 6 bis 22 Kohlenstoffatomen, R³ für Wasserstoff oder eine Methylgruppe und m für Zahlen von 1 bis 10 steht. Typische Beispiele sind Anlagerungsprodukte von 1 bis 10 Mol Ethylenoxid und/oder 1 bis 2 Mol Propylenoxid an Fettalkohole wie z.B. Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Anlagerungsprodukte von durchschnittlich 1 bis 5 Mol Ethylenoxid bzw. 1,2 Mol Propylenoxid und 1 bis 3 Mol Ethylenoxid an technische Kokosfettalkohole mit 8 bis 18 und insbesondere 12 bis 14 Kohlenstoffatomen. Die Alkoxylate können dabei sowohl eine konventionell breite als auch eine eingeengte Homologenverteilung aufweisen.

### Sulfatierung

Die Sulfatierung der Mischungen mit gasförmigem Schwefeltrioxid kann in der für Fettsäureniedrigalkylester bekannten Weise **[J.Falbe (ed.), "Surfactants in consumer products", Springer Verlag, Berlin-Heidelberg, 1987, S. 61]** erfolgen, wobei Reaktoren, die nach dem Fallfilmprinzip arbeiten, bevorzugt sind. Dabei wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt. Das Einsatzverhältnis des gasförmigen Schwefeltrioxids beträgt vorzugsweise pro Mol zu sulfatierender Mischung (a+b) 0,95 bis 1,5, vorzugsweise jedoch 1,0 bis 1,3 Mol Schwefeltrioxid. Die Sulfatierung wird vorzugsweise bei Temperaturen von 40 bis 85°C, insbesondere jedoch 10 bis 20°C oberhalb des Schmelzpunktes der Mischungen durchgeführt.

### Neutralisation und Bleiche

Die bei der Reaktion anfallenden sauren Sulfierprodukte werden in wäßrige Basen eingerührt, neutralisiert und auf einen pH-Wert von 8 bis 9 eingestellt. Als Basen für die Neutralisation kommen Alkalimetallhydroxide wie Natrium-, Kalium- und Lithiumhydroxid, Erdakalimetalloxide und -hydroxide wie Magnesiumoxid, Magnesiumhydroxid, Calciumoxid und Calciumhydroxid, Ammoniak, Mono-, Di- und Tri-C₂₋₄-Alkanolamine, beispielsweise Mono-, Di- und Triethanolamin sowie primäre, sekundäre oder tertiäre C₁₋₄-Alkylamine in Betracht. Die Neutralisationsbasen gelangen dabei vorzugsweise in Form 5 bis 55 Gew.-%iger wäßriger Lösungen zum Einsatz, wobei 10 bis 25 Gew.-%ige wäßrige Natriumhydroxidlösungen bevorzugt sind.

Die Sulfierprodukte können nach Neutralisation in an sich bekannter Weise durch Zusatz von Wasserstoffperoxid- oder Natriumhypochloritlösung gebleicht werden. Dabei werden, bezogen auf den Feststoffgehalt in der Lösung der Sulfierprodukte, 0,2 bis 2 Gew.-% Wasserstoffperoxid, berechnet als 100 Gew.-%ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

### Gewerbliche Anwendbarkeit

Die erfindungsgemäßen anionischen Detergensgemische zeichnen sich durch ein starkes Schaum- und hohes Reinigungsvermögen sowie eine gute hautkosmetische Verträglichkeit aus. Ein weiterer Gegenstand der Erfindung betrifft daher ihre Verwendung zur Herstellung von oberflächenaktiven Mitteln, wie beispielsweise Wasch-, Spül- und Reinigungsmitteln sowie Produkten zur Reinigung und Pflege von Haut und Haaren, in denen sie in Mengen von 1 bis 35, vorzugsweise 5 bis 25 Gew.-% - bezogen auf die Mittel - enthalten sein können.

### Beispiele

### Beispiele 1 bis 3, Vergleichsbeispiel V1

Mischungen von jeweils 1 Mol Laurinsäuremonoethanolamid (A) und Kokosfettalkohol+2EO-Addukt (B) wurden und bei einer Temperatur (TR) ca. 15°C oberhalb ihres Schmelzpunktes (FP) in einem Fallfilmreaktor mit 1,15 Mol gasförmigem Schwefeltrioxid umgesetzt. Das Schwefeltrioxid wurde durch Erhitzen aus einer entsprechenden Menge 65 Gew.-%igen Oleums ausgetrieben und auf eine Konzentration von 3 Vol.-% verdünnt. Nach der Sulfatierung wurden die sauren Reaktionsprodukte in wäßrige 10 Gew.-%ige Natriumhydroxidlösung eingerührt und der pH-Wert mit 50 Gew.-%iger Natriumhydroxidlösung stetig nachgeregelt. Die Endprodukte wurden auf pH = 8,5 und einen Waschaktivgehalt von ca. 30 % eingestellt. Der Aniontensidgehalt (WAS) sowie die unsulfierten Anteile (US) wurden nach den DGF-Einheitsmethoden, Stuttgart 1950-1984, H-III-10 und G-II-6b ermittelt. Die Ergebnisse sind in Tabelle 1 zusammengefaßt.

**Tabelle 1**

| **Sulfatierung von Laurinsäureethanolamid/Ethoxylat-Compounds** | | | | | | | | | |
|---|---|---|---|---|---|---|---|---|---|
| **Bsp.** | **A:B** | **FP** **°C** | **T** **°C** | **Sulfatierungsprodukt** | | | | | |
| | | | | **WAS** **%** | **US** **%** | **Sulfat** **%** | **H**_{**2**}**O %** | **S°** **%** | **Oxaz.** **%** |
| 1 | 20:80 | 39 | 50 | 29,0 | 1,5 | 0,9 | 68,6 | 93,4 | 3,0 |
| 2 | 50:50 | 57 | 70 | 29,9 | 1,7 | 2,1 | 66,7 | 92,6 | 4,1 |
| 3 | 70 :30 | 64 | 82 | 26,9 | 3,3 | 3,8 | 66,0 | 85,4 | 4,8 |
| V1* | 100:0 | 73 | 95 | - | - | - | - | - | - |

| | | | | | | | | | |
|---|---|---|---|---|---|---|---|---|---|
| **Legende:** WAS = Waschaktivgehalt | | | | | | | | | |
| US = Unsulfierter Anteil | | | | | | | | | |
| Sulfat = Natriumsulfat | | | | | | | | | |
| S° = Sulfiergrad | | | | | | | | | |
| Oxaz. = Oxazolingehalt, bezogen auf 100 Gew.-% Laurinsäureethanolamidsulfat | | | | | | | | | |
| * = kristallisiert im Reaktor, nicht sulfatierbar | | | | | | | | | |

## Patentansprüche

1. Anionische Detergensgemische, dadurch erhältlich, daß man Mischungen von
(a) Fettsäurealkanolamiden mit 6 bis 22 Kohlenstoffatomen im Fettsäurerest und
(b) Fettalkoholpolyalkylenglycolen mit 6 bis 22 Kohlenstoffatomen im Fettalkoholrest
im Gewichtsverhältnis 5 : 95 bis 70 : 30 in an sich bekannter Weise gemeinsam sulfatiert und anschließend mit wäßrigen Basen neutralisiert.

2. Verfahren zur Herstellung von anionischen Detergensgemischen, bei dem man Mischungen von
(a) Fettsäurealkanolamiden mit 6 bis 22 Kohlenstoffatomen im Fettsäurerest und
(b) Fettalkoholpolyalkylenglycolen mit 6 bis 22 Kohlenstoffatomen im Fettalkoholrest
im Gewichtsverhältnis 5 : 95 bis 70 : 30 in an sich bekannter Weise gemeinsam sulfatiert und anschließend mit wäßrigen Basen neutralisiert.

3. Verfahren nach Anspruch 2, **dadurch gekennzeichnet**, daß man Fettsäurealkanolamide der Formel **(I)** einsetzt,
R¹CO-NH(CH₂)ₙOH (I)
in der R¹CO für einen linearen oder verzweigten, gesättigten und/oder ungesättigten Acylrest mit 6 bis 22 Kohlenstoffatomen und n für Zahlen von 2 bis 4 steht.

4. Verfahren nach den Ansprüchen 2 und 3, **dadurch gekennzeichnet**, daß man Fettalkoholalkylengylcolether der Formel **(II)** einsetzt, 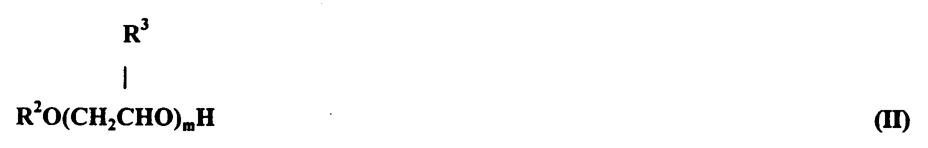 in der R² für einen linearen Alkyl- und/oder Alkenylrest mit 6 bis 22 Kohlenstoffatomen, R³ für Wasserstoff oder eine Methylgruppe und m für Zahlen von 1 bis 10 steht.

5. Verfahren nach den Ansprüchen 2 bis 4, **dadurch gekennzeichnet,** daß man die Sulfatierung in Fallfilmreaktoren mit gasförmigem Schwefeltrioxid durchführt, das mittels eines Inertgases aufeine Konzentration von 1 bis 8 Vol.-% verdünnt wird.

6. Verfahren nach den Ansprüchen 2 bis 5, **dadurch gekennzeichnet**, daß man pro Mol zu sulfatierender Mischung (a+b) 0,95 bis 1,5 Mol Schwefeltrioxid einsetzt.

7. Verfahren nach den Ansprüchen 2 bis 6, **dadurch gekennzeichnet**, daß man die Sulfatierung bei Temperaturen im Bereich von 40 bis 85°C durchführt.

8. Verfahren nach den Ansprüchen 2 bis 7, **dadurch gekennzeichnet**, daß man die Neutralisation mit 5 bis 55 Gew.-%igen wäßrigen Basen aus der von Alkalimetallhydroxiden, Erdalkalimetalloxiden und -hydroxiden, Ammoniak, Mono-, Di- und Tri-C₂₋₄-Alkanolaminen sowie primären, sekundären und tertiären C₁₋₄-Alkylaminen gebildeten Gruppe durchführt.

9. Verwendung von anionischen Detergensgemischen nach Anspruch 1 zur Herstellung von oberflächenaktiven Mitteln.

## Claims

1. Anionic detergent mixtures obtainable by sulfating mixtures of
(a) fatty acid alkanolamides containing 6 to 22 carbon atoms in the fatty acid component and
(b) fatty alcohol polyalkylene glycols containing 6 to 22 carbon atoms in the fatty alcohol component
in a ratio by weight of 5:95 to 70:30 together by methods known per se and then neutralizing the products obtained with aqueous bases.

2. A process for the production of anionic detergent mixtures in which mixtures of
(a) fatty acid alkanolamides containing 6 to 22 carbon atoms in the fatty acid component and
(b) fatty alcohol polyalkylene glycols containing 6 to 22 carbon atoms in the fatty alcohol component
in a ratio by weight of 5:95 to 70:30 are sulfated together by methods known per se and the products obtained are neutralized with aqueous bases.

3. A process as claimed in claim 2, characterized in that fatty acid alkanolamides corresponding to formula (I):
R¹CO-NH(CH₂)ₙOH
in which R¹CO is a linear or branched, saturated and/or unsaturated acyl group containing 6 to 22 carbon atoms and n is a number of 2 to 4, are used.

4. A process as claimed in claims 2 and 3, characterized in that fatty alcohol alkyleneglycol ethers corresponding to formula (II): 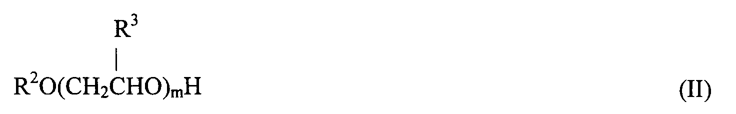 in which R² is a linear alkyl and/or alkenyl group containing 6 to 22 carbon atoms, R³ is hydrogen or a methyl group and m is a number of 1 to 10, are used.

5. A process as claimed in claims 2 to 4, characterized in that the sulfation reaction is carried out in falling film reactors with gaseous sulfur trioxide diluted with an inert gas to a concentration of 1 to 8% by volume.

6. A process as claimed in claims 2 to 5, characterized in that 0.95 to 1.5 moles sulfur trioxide are used per mole of mixture (a+b) to be sulfated.

7. A process as claimed in claims 2 to 6, characterized in that the sulfation reaction is carried out at temperatures of 40 to 85°C.

8. A process as claimed in claims 2 to 7, characterized in that the neutralization step is carried out with 5 to 55% by weight aqueous bases from the group consisting of alkali metal hydroxides, alkaline earth metal oxides and hydroxides, ammonia, mono-, di- and tri-C₂₋₄-alkanolamines and primary, secondary and tertiary C₁₋₄ alkyl amines.

9. The use of the anionic detergent mixtures claimed in claim 1 for the production of surface-active compositions.

## Revendications

1. Mélanges de détergents anioniques, obtenables en sulfatant conjointement d'une manière connue en soi des mélanges
(a) d'alcanolamides d'acides gras, dont le radical acide gras comporte 6 à 22 atomes de carbone et
(b) de polyalkylèneglycols d'alcools gras, dont le radical alcool gras possède 6 à 22 atomes de carbone,
dans un rapport pondéral de 5:95 à 70:30, et on les neutralise ensuite à l'aide de bases aqueuses.

2. Procédé de production de mélanges de détergents anioniques, dans lequel on sulfate conjointement d'une manière connue en soi des mélanges
(a) d'alcanolamides d'acides gras, dont le radical acide gras comporte 6 à 22 atomes de carbone et
(b) de polyalkylèneglycols d'alcools gras, dont le radical alcool gras possède 6 à 22 atomes de carbone,
dans un rapport pondéral de 5:95 à 70:30, et on les neutralise ensuite à l'aide de bases aqueuses.

3. Procédé selon la revendication 2, **caractérisé en ce que** l'on met en oeuvre des alcanolamides d'acides gras de la formule **(I)**,
R¹CO-NH(CH₂)ₙOH (I)
dans laquelle R¹CO représente un radical acyle linéaire ou ramifié, saturé et/ou insaturé comportant 6 à 22 atomes de carbone et n correspond à un nombre de 2 à 4.

4. Procédé selon les revendications 2 et 3, **caractérisé en ce que** l'on met en oeuvre des polyalkylèneglycoléthers d'alcools gras de la formule **(II),** 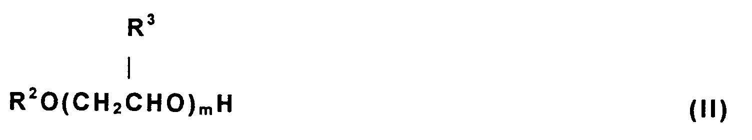 dans laquelle R² représente un radical alkyle et/ou alcényle comportant 6 à 22 atomes de carbone, R³ correspond à l'hydrogène ou à un groupe méthyle et m est un nombre de 1 à 10.

5. Procédé selon les revendications 2 à 4, **caractérisé en ce que** l'on opère la sulfatation dans des réacteurs à ruissellement avec du trioxyde de soufre gazeux, qui est dilué à l'aide d'un gaz inerte à une concentration de 1 à 8 % en volume.

6. Procédé selon les revendications 2 à 5, **caractérisé en ce que** l'on met en oeuvre 0,95 à 1,5 mol de trioxyde de soufre par mol du mélange à sulfater (a+b).

7. Procédé selon les revendications 2 à 6, **caractérisé en ce que** l'on opère la sulfatation à des températures comprises dans l'intervalle de 40 à 85 °C.

8. Procédé selon les revendications 2 à 7, **caractérisé en ce que** l'on opère la neutralisation avec des bases aqueuses à 5 à 55 % en poids faisant partie du groupe formé des hydroxydes de métaux alcalins, des oxydes et des hydroxydes de métaux alcalino-terreux, de l'ammoniac, des mono-, des di- et des trialcanolamines en C₂₋₄, ainsi que des alkylamines primaires, secondaires et tertiaires en C₁₋₄.

9. Utilisation des mélanges de détergents anioniques selon la revendication 1 pour la production de produits tensioactifs.
